# EUROPEAN PATENT SPECIFICATION

(11) **EP 1 601 736 B1**
(45) Date of publication and mention of the grant of the patent: **28.05.2008**
(21) Application number: 04709092.3
(22) Date of filing: 06.02.2004
(51) Int. Cl.: C09J 7/02, B65D 33/18, B65D 63/10, A61F 13/58

(54) **CLOSURE SYSTEMS WITH SELF-ADHERING ADHESIVES, AND ARTICLES**
VERSCHLUSSSYSTEME MIT SELBSTHAFTENDEN KLEBSTOFFEN SOWIE GEGENSTÄNDE
SYSTEMES DE FERMETURE AVEC AUTO-ADHESIFS, ET ARTICLES

(30) Priority: 13.03.2003 US 388037
(43) Date of publication of application: 07.12.2005
(73) Proprietor: 3M Innovative Properties Company, St. Paul, MN 55133-3427 (US)
(72) Inventor: EVERAERTS, Albert, I., Saint Paul, Minnesota 55133-3427 (US); MA, Jingjing, Saint Paul, Minnesota 55133-3427 (US); WANG, Shou-Lu, G., Saint Paul, Minnesota 55133-3427 (US)
(74) Representative: Vossius & Partner
(86) International application number: PCT/US2004/003555
(87) International publication number: WO 2004/083331

(56) References cited:
- EP-A- 0 431 391
- US-A- 4 785 940
- US-A- 4 942 195
- US-A- 5 378 536
- US-A- 5 733 652

## Description

Mechanical fastener (i.e., closure) systems are widely used for a variety of applications where quick close and quick release are desirable. These include hook and loop fasteners and mushroom-shaped fasteners. Although such systems are widely used and have been shown to be extremely useful, they have certain undesirable characteristics. For example, they can snag fabrics, they collect lint and dust, they do not have a very thin profile, and they do not provide a very good seal against the transport of fluids.

US-A-5,378,536 discloses a certain repositionable adhesive tape.

Adhesive-based fastener systems are known, including substantially non-tacky (i.e., low-tack and tack-free) contact responsive fastener systems. Non-tacky adhesive fastener systems are desirable because they do not generally adhere to other materials. However, conventional non-tacky fastener systems are often not capable of forming a seal quickly under relatively low to moderate pressure, with relatively high shear and peel values. Furthermore, many of the ingredients of conventional non-tacky adhesive fastener systems are not approved for skin contact and are relatively expensive. Thus, there is a need for alternative adhesive-based fastener systems.

In the present invention closure systems containing self-adhering adhesives are employed. In one embodiment, the present invention provides a method for closing a closure system that includes: at least two opposing closure elements that are capable of overlapping to form an adhesive closure; and a self-adhering adhesive composition on each of the closure elements; wherein the self-adhering adhesive composition includes: 50 wt-% to 90 wt-% of an adhesive elastomeric material comprising a nonpolar elastomer; 10 wt-% to 40 wt-% of a wax; 0 to less than 5 wt-% of a plasticizer; wherein the percentages are based on the total weight of the adhesive elastomeric material plus wax plus optional plasticizer; all wherein the self-adhering adhesive composition has a T-peel of at least-100 grams per cm and a loop tack to steel of no greater than 40 grams per square centimeter, wherein the T-peel is measured according to the T Peel Adhesion Test and the loop tack to steel is measured according to the Loop Tack Steel Adhesion Test, wherein the method comprises the step of bringing the opposing closure elements into contact to yield an adhesive closure.

The closure system is preferably for a disposable diaper, although it can also function as a closure for a bag (e.g., a food bag), a banding system or tape, or a variety of other articles. Such articles include, for example, at least two opposing closure elements at separate locations on the article that are capable of overlapping to form an adhesive closure; and a self-adhering adhesive composition on each of the closure elements as described herein.

The present invention also provides a bag that includes: at least two regions of the bag having a layer of a self-adhering adhesive composition disposed thereon at separate locations in a manner such that the regions are capable of overlapping to form an adhesive closure; wherein the self-adhering adhesive composition includes: 50 wt-% to 90 wt-% of an adhesive elastomeric material comprising a nonpolar elastomer, 10 wt-% to 40 wt-% of a wax; 0 to less than 5 wt-% of a plasticizer; wherein the percentages are based on the total weight of the adhesive elastomeric material plus wax plus optional plasticizer, and wherein the self-adhering adhesive composition has a T-peel of at least 100 grams per cm and a loop tack to steel of no greater than 40 grams per square centimeter, wherein the T-peel is measured according to the T Peel Adhesion Test and the loop tack to steel is measured according to the Loop Tack Steel Adhesion Test.

The present invention also provides a banding system that includes at least two strips of a banding material with at least two regions of a self-adhering adhesive, composition disposed thereon at separate locations in a manner such that the regions of adhesive are capable of overlapping to form an adhesive closure. The self-adhering adhesive composition includes: 50 wt-% to 90 wt-% of an adhesive elastomeric material comprising a nonpolar elastomer; 10 wt-% to 40 wt-% of a wax; 0 to less than 5 wt-% of a plasticizer; wherein the percentages are based on the total weight of the adhesive elastomeric material plus wax plus optional plasticizer, and wherein the self-adhering adhesive composition has a T-peel of at least 100 grams per cm and a loop tack to steel of no greater than 40 grams per square centimeter, wherein the T-peel is measured according to the T Peel Adhesion Test and the loop tack to steel is measured according to the Loop Tack Steel Adhesion Test.

The present invention also provides an apparel comprising a closure system, the closure system comprising at least two opposing closure elements that are capable of overlapping to form an adhesive closure; and a self-adhering adhesive composition on each of the closure element. The self-adhering adhesive composition comprises 50 wt-% to 90 wt-% of an adhesive elastomeric material comprising a nonpolar elastomer; 10 wt-% to 40 wt-% of a wax; and 0 to less than 5 wt-% of a plasticizer; wherein the percentages are based on the total weight of the adhesive elastomeric material plus wax plus optional plasticizer. The self-adhering adhesive composition has a T-peel of at least 100 grams per centimeter and a loop tack to steel of no greater than 40 grams per square centimeter, wherein the T-peel is measured according to the T Peel Self-Adhesion Test and the loop tack to steel is measured according to the Loop Tack Steel-Adhesion Test. The closure system is to be used in close proximity to the wearer.

As used herein, "a," "an," "the," "at least one," and "one or more" are used interchangeably. Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments.
FTG. 1 is a schematic view of a disposable diaper as it would appear while being worn by a person.
FIG. 2 is a schematic view of one embodiment of a banding system being used to band a bunch of vegetables.
FIG. 3 is a schematic view of one embodiment of a banding tape being used to band a bunch of vegetables.
FIG. 4 is a schematic view of a flexible plastic bag with a self-adhering adhesive of the present invention.

In the present invention closure systems containing self-adhering adhesives are employed. Herein, a "self-adhering" adhesive is one that will adhere to itself and others within the class of adhesives described herein, but not to many other materials. Although such adhesives may wet out on other surfaces and form adhesive bonds, they are most useful in applications in which they adhere to each other. That is, whether or not adhesives used in the present invention are low-tack or tack-free, pieces of tape bearing the adhesive have a remarkable ability to form bonds to each other.

Various adhesives used in the present invention have one or more of the following properties (as defined herein): high peel; high shear; quick stick; and low thumb tack. Particularly desirable adhesives have all of these properties.

Useful adhesives for the present invention have a T-peel of at least 100 grams per centimeter (cm), at least 200 grams per cm for certain embodiments, and at least 300 grams per cm for other embodiments. Typically, adhesives with higher T-peel values are desirable. Herein, these T-Peel values are self-adhesion values. They are a measure of the force to peel a tape adhered to itself using ASTM D-1876 under the protocol described in the Examples Section.

Useful adhesives for the present invention have a shear adhesion value of at least 100 minutes for certain embodiments, at least 500 minutes for other embodiments, and at least 900 minutes for other embodiments. Typically, adhesives with higher shear adhesion values are desirable. Herein, these shear adhesion values are a measure of the force to shear a tape adhered to itself using the protocol described in the Examples Section.

As used herein, "quick stick" adhesive is one that will adhere to itself ("Loop Tack Self-Adhesion) with a very short dwell time. This can be measured using ASTM D-6195 Method A as described in the Examples Section.

Useful adhesives for the present invention have a loop tack self-adhesion of at least 120 grams per square centimeter (g/cm²) for certain embodiments, at least 150 grams per square centimeter for other embodiment, and at least 200 grams per square centimeter for other embodiments. Typically, adhesives with higher loop tack self-adhesion values are desirable.

Useful adhesives for the present invention have a loop tack steel-adhesion of no greater than 40 grams per square centimeter, and no greater than 80 grams per square centimeter for certain embodiments. Typically, adhesives with lower loop tack steel-adhesion values are desirable.

Useful adhesives, for the present invention have a thumb tack of no greater than 3 for certain embodiments. Thumb tack can be measured using the method described in the Examples Section. Typically, adhesives with lower thumb tack values are desirable.

Certain useful adhesives for the present invention are refastenable. This means that surfaces containing the self-adhering adhesive described herein can be adhered together, pulled apart, and readhered with similar adhesion values (e.g., T-peel self adhesion).

Useful self-adhering adhesives for the invention can be economically converted to tapes by being coated onto backings at high speeds without the use of solvents, or can be coated from solution when the components can be solubilized in a common solvent or solvent mixture. Depending on the backing, the backings can be surface treated to promote adhesion of the adhesive thereto. The resulting tapes can be marketed in strips or in wide sheets and usually have flexible backings for ease of storage, handling, and application.

The backings can be films, foams, nonwovens, or woven materials. They can be totally or partially planar or structured, such as with hooks, loops, etc. For example, the backing of one closure element comprises hooks and the backing of one closure element comprises loops. The adhesive can at least partially overlap the structured portions of the backing or be adjacent thereto.

Certain adhesives useful in the present invention include components that are approved for contact with skin. Thus, certain of these adhesives are well suited for apparel closures that are used in close proximity to the wearer. Examples of uses for such closure systems include diapers, incontinence devices, surgical gowns, hats, booties, clean room garments, ankle bands, wrist bands, and the like. When used, as in apparel closures, the adhesive can be directly applied to the article or applied as a tape where the side that is permanently attached to the article can have a suitable conventional adhesive.

Closure systems used in the present invention are also useful for non-apparel applications. For example, the closure systems can be used for bags such as dog food bags or other food bags as well as nonfood bags, etc., as well as banding systems and tapes for bundling vegetables, for example.

Typically, in a closure system the adhesive would generally be applied as patches, either by stitching or using an adhesive, on each side of a closure point, e.g., on opposing faces of at least two closure elements that mate to complete the closure. When the two closure elements of the article are brought into contact, the adhesive patches will come into contact yielding an adhesive closure. The patches can be of a size and arrangement such that they will contact each other over a number of overlapping positions of the closure elements to form an adjustable closure.

In a specific embodiment, the self-adhering adhesives can be used to provide a disposable diaper by applying a patch of the adhesive to the inner face of each of the corners at the back of a disposable diaper and also to the outer shell where the front of the diaper can be overlapped by the corners when the diaper is wrapped around the waist of a person. The face-to-face contact between those adhesive patches holds the diaper securely in place. For certain embodiments, the patches can be easily peeled apart to remove the diaper and be resealed. For other embodiments, the patches are not easily peeled apart and cannot be resealed.

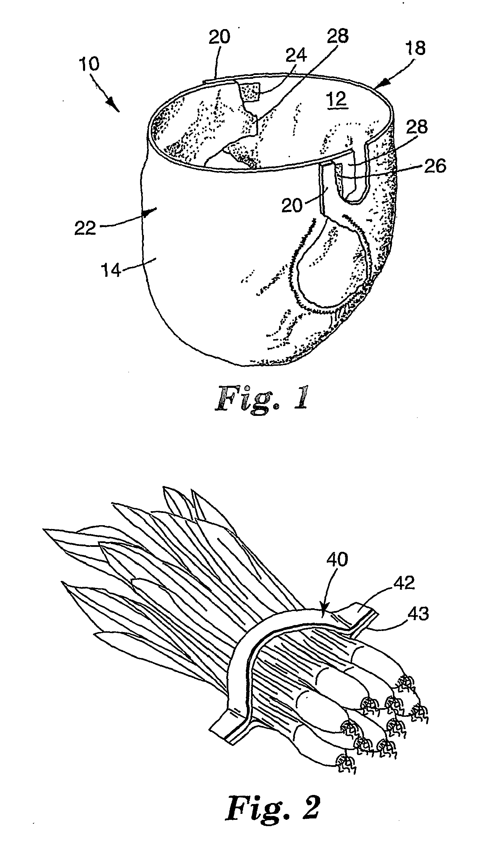

The disposable diaper shown in FIG. 1 is a conventional three layer composite including a liquid-permeable user-contacting topsheet 12, a liquid-impervious outer shell (backsheet) 14, and an absorbent layer therebetween (not shown). At the back 18 of the diaper are corners 20 that overlap the front of the diaper 22 at corners 28 when the diaper is worn as shown. On the topsheet side of each corner 20 is placed a patch of a self-adhering adhesive described herein 24. On the outer shell 14 at the front 22 of the diaper are two patches 26 of a self-adhering adhesive described herein. The patches are placed on the diaper such that they will come into contact when the diaper is worn as shown.

The overlapping portions of the adhesive patches provide a force to peel (i.e., a T-Peel) of at least 100 grams per centimeters. To provide this peel force resistance and still provide for adjustability, the adhesive patches are generally placed as is shown in FIG. 1. Each patch is typically of a size of at least 3 centimeters (cm) in length and often up to 6 cm in length. Each patch is typically of a size of at least 1.5 cm in width and often up to 3 cm in width. The lengthwise directions of the patches are preferably orthogonal to each other. This orientation will provide the greatest degree of adjustability for the preferred patches. Where the patches are so oriented and overlap over an entire width portion, the area of contact is typically at least 1.75 cm², and often up to 9 cm², for rectilinear shaped patches. Generally this degree of overlap provides sufficient peel resistance for use as a diaper closure system while avoiding excessive or wasteful use of the adhesive.

Closure systems used in the present invention can also be used in banding systems and on banding tapes. Such articles are used to bundle a variety of items such as vegetables (e.g., broccoli, green onions, carrots, celery, etc.), flowers, straws, pens, pencils, newspapers, as well as numerous others. Examples of banding systems and tapes are disclosed in U.S. Pat. No. 5,733,652.

In one embodiment, a banding system 40, as shown in FIG. 2, includes at least two strips of a banding material with at least two regions having a layer of a self-adhering adhesive disposed thereon at separate locations in a manner such that the regions of adhesive are capable of overlapping to form an adhesive closure. Specifically, the banding system 40 includes two strips 42 and 43 in aligned contacting relation. A first strip 42 has first and second ends and has disposed thereon a layer of a self-adhering adhesive as described herein. A second strip 43 has first and second ends and has disposed thereon a layer of a self-adhering adhesive as described herein. The first ends of the first and second strips are capable of being bonded together and the second ends of the first and second strips are capable of being joined (e.g., bonded together) by the self-adhering adhesive. The first and second strips can be made of a wide variety of materials, such as an elastic polymeric material as disclosed in U.S. Pat. No. 5,733,652.

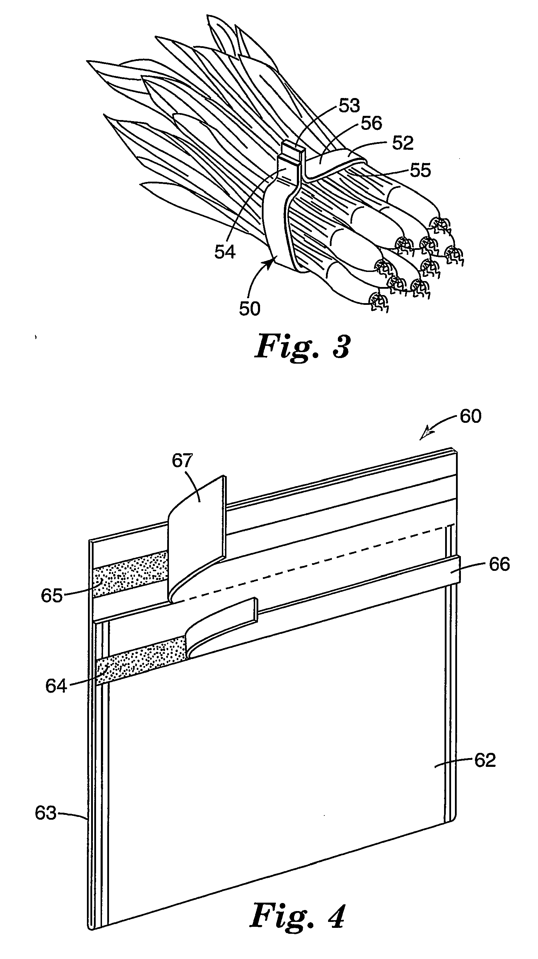

Referring to FIG. 3, in another embodiment, a banding tape 50 includes a strip of material 52 (e.g., elastic polymeric material) having first and second ends (53 and 54) and an inner surface 55 and an outer surface 56. A self-adhering adhesive described herein is disposed in at least one region (e.g. a continuous layer or discrete regions) on at least a portion of each of said surfaces (not shown) or on at least a portion of the same surface (as shown in FIG. 3) in a manner such that the tapes are capable of being joined (e.g., bonded together) by the self-adhering adhesive. The strip of banding material can be made of a wide variety of materials, such as an elastic polymeric material as disclosed in U.S. Pat. No. 5,733,652.

FIG. 4 shows a representative embodiment of a flexible plastic bag 60 with at least two regions of the bag having a layer of a self-adhering adhesive composition disposed thereon at separate locations in a manner such that the regions are capable of overlapping to form an adhesive closure. Specifically, bag 60 includes a front panel 62, back panel 63, two closure strips 64 and 65 (i.e., layers of an adhesive coated in strips) of a self-adhering adhesive of the present invention (one strip on each of the panels), with removable liners 66 and 67 applied to the strips prior to use. The liners of both strips may be removed and the adhesive closure strips 64 and 65 pressed together to form a mutual bond. Such bags are disclosed for example, in U.S. Pat. No. 4,785,940.

These embodiments of various articles are exemplary only. Various other embodiments of these and other articles are within the scope of the present invention.

### ELASTOMERIC MATERIALS AND TACKIFYING RESINS

Elastomeric materials may possess adhesive characteristics; however, many use one or more tackifying resins to enhance their adhesive characteristics. Thus, "adhesive elastomeric materials" as used herein include one or more elastomers optionally with one or more tackifying resins, plasticizers, and other additives.

Elastomeric materials (i.e., elastomers) are materials that at room temperature return rapidly to approximately their initial dimensions and shape after substantial deformation by a weak stress and release of the stress. Elastomers are among the group of polymers that can easily undergo very large, reversible elongations (often up to 500% and more often up to 1000%) at relatively low stresses.

The elastomeric materials may be classified as thermoplastic elastomers. Thermoplastic elastomeric materials are generally defined as materials that behave as elastomers at ambient temperatures, but are thermoplastic at elevated temperatures where they can be molded and remolded.

Elastomeric materials useful in the present invention are nonpolar, and typically hydrocarbon-based. They include, but are not limited to, natural rubbers, butyl rubbers, synthetic polyisoprenes, ethylene-propylenes, polybutadienes, polyisobutylenes, linear, branched, radial, star, and tapered block copolymers such as styrene-isoprene, styrene-ethylene-butylene, styrene-ethylene-propylene, and styrene-butadiene block copolymers, poly-alpha-olefin based thermoplastic elastomeric materials such as those represented by the formula -(CH₂-CHR)- where R is an alkyl group containing 2 to 10 carbon atoms, and poly-apha-olefins based on metallocene catalysis.

For certain embodiments, the desired elastomeric material includes thermoplastic block copolymers, particularly those containing styrene in the hard block.

The elastomeric materials may be used in various combinations and various amounts as desired, which can be readily determined by one of skill in the art. Typically, the amounts of the various components of the adhesive compositions of the present invention are based on 100 parts of the elastomeric material, whether this is one elastomer of a mixture of elastomers.

Useful examples of tackifying resins suitable for enhancing the adhesive characteristics of the elastomeric material include, but are not limited to, aliphatic hydrocarbon resins, aromatic hydrocarbon resins, aromatic modified aliphatic hydrocarbon resins, rosin, natural resins such as dimerized or hydrogenated balsams and esterified abietic acids, polyterpenes, terpene phenolics, phenol-formaldehyde resins, dicyclopentadiene resins, and rosin esters. These tackifying resins may be hydrogenated to improve color and stability.

Tackifying resins can be used in various combinations and amounts as desired, which can be readily determined by one of skill in the art. Typically, a total amount of no more than 300 parts of one or more tackifying resins, per 100 parts of elastomeric material, is included in an adhesive composition used in the present invention. For certain embodiments of adhesive compositions used in the present invention no tackifying resin is included or only up to 110 parts (total amount) per 100 parts of the elastomeric material is included.

Adhesive elastomeric materials, as used herein, include one or more elastomers and optionally one or more tackifying resins. Typically, a total amount of at least 60 percent by weight (wet-%) of adhesive elastomeric material, wherein the percentage is based on the total weight of the adhesive elastomeric material plus wax plus optional plasticizer, is included in an adhesive composition of the present invention. A total amount of no more than 90 wt-% of adhesive elastomeric material is included, wherein the percentage is based on the total weight of the adhesive elastomeric material plus wax plus optional plasticizer.

### WAXES

Useful adhesive compositions for the present invention include one or more waxes. Typically, a wax is defined as a material that has a molecular weight below its critical entanglement molecular weight. Useful waxes for the present invention are nonpolar and may be of either natural or synthetic origin.

Natural waxes include those derived from insects, animals, and plants, for example. Synthetic waxes can be derived from petroleum or coal products. Petroleum waxes are conventionally subdivided into paraffin waxes and microcrystalline waxes, being obtained at different stages in the process of refining crude oil. Synthetic waxes can be derived from the polymerization of hydrocarbon monomers such as ethylene, propylene, and the like. Synthetic waxes can be made by various approaches including high pressure polymerization, low pressure polymerization using Ziegler-Natta type catalysts, or the thermal degradation of high molecular weight polymers such as polyethylene and polypropylene. Synthetic waxes include polyethylene waxes, Fischer-Tropsch waxes (polymethylene waxes), and chemically modified hydrocarbon waxes (i.e., microcrystalline, polyethylene and polymethylene waxes that have been chemically modified to introduce particular properties).

In general waxes can possess a wide variety of molecular weights. For certain embodiments, however, preferred waxes are those having a molecular weight of less than 1500. Such molecular weights are number average molecular weights.

Waxes can be used in various combinations and amounts as desired, which can be readily determined by one of skill in the art. Typically, for certain embodiments of the present invention, a total amount of no more than 30 wt-% of one or more waxes is included in an adhesive composition of the present invention. For certain other embodiments, a total amount of no more than 35 wt-% of one or more waxes is included in an adhesive composition of the present invention. A total amount of no more than 40 weight percent (wt-%) of one or more waxes is included in an adhesive composition for the present invention. A total amount of at least 10 wt-% of one or more waxes is included. These percentages are based on the total weight of the adhesive elastomeric material plus wax plus optional plasticizer based on the total weight of the adhesive composition.

### PLASTICIZERS

Adhesive compositions for the present invention can include one or more plasticizers if desired, although certain embodiments of the present invention do not include plasticizers. Generally, the plasticizing agents (i.e., plasticizers) can be liquid or solid at room temperature, have a range of molecular weights and architectures, and are compatible with the base adhesive elastomeric material. Additionally, mixtures of solid and liquid, monomeric and polymeric, and other combinations of plasticizing agents can be used in the present invention. The plasticizing agents can be either polymeric or monomeric in nature. Typically, monomeric plasticizing agents are derived from low molecular weight acids or alcohols, which are then esterified with respectively a monofunctional alcohol or monofunctional acid. Examples of these include, but are not limited to, esters of mono- and multibasic acids, such as isopropyl myristate, dibutyl phthalate, diisoctyl phthalate, and dibutyl adipate, dibutylsebacate. Useful polymeric plasticizing agents are typically derived from cationically or free-radically polymerizable, condensation polymerizable or ring-opening polymerizable monomers to make low molecular weight polymers. Other examples of polymeric plasticizing agents can include polybutene, liquid polyisoprenes, and amorphous polyolefins. Particularly useful examples of plasticizers include, but are not limited to, polybutene, paraffinic oils, naphthenic oils, petrolatum, and certain phthalates with long aliphatic side chains such as ditridecyl phthalate.

Plasticizers can be used in various combinations and amounts as desired, which can be readily determined by one of skill in the art. For certain embodiments of adhesive compositions for the present invention no plasticizer is included or only up to 5 wt-% (total amount) of the elastomeric material is included, based on the total weight of the adhesive elastomeric material plus wax plus optional plasticizer.

### OPTIONAL ADDITIVES

Additives such as pigments, fillers, medicinals (e.g., antimicrobials and other biologically active agents), crosslinkers, and antioxidants may be used in the adhesive compositions used in the present invention. Pigments and fillers may be incorporated into the adhesive blend in order to manipulate the properties of the adhesive according to its intended use. Examples of fillers include, but are not limited to, inorganic fillers such as zinc oxide, alumina trihydrate, talc, titanium dioxide, aluminum oxide, and silica. Crosslinkers may be activated chemically or by radiation. Radiation crosslinkers such as benzophenone, derivatives ofbenzophenone, and substituted benzophenones may be added to the adhesive compositions of the invention. Antioxidants may be used to protect against severe environmental aging caused by ultraviolet light or heat. Examples of antioxidants include, but are not limited to, hindered phenols, amines, and sulfur and phosphorous hydroxide decomposes.

### EXAMPLES

Objects and advantages of this invention are further illustrated by the following examples, but the particular materials and amounts thereof recited in these examples, as well as other conditions and details, should not be construed to unduly limit this invention.

### Test Methods

Tapes utilizing the adhesives of the invention and a variety of backings, were tested with one or more of the following test methods.

### T Peel Self-Adhesion Test

The force to peel a tape adhered to itself was measured using ASTM D-1876 under the following protocol. Two pieces (2.54 cm wide by 15.24 cm long) of the same tape were cut and laminated to each other adhesive side to adhesive side with approximately 7.62 cm of overlap. A 100-gram hard rubber roller was passed over the laminate once in each direction at 30.5 cm per minute to firmly adhere the two tapes to each other. The two-tape laminate was then tested within 10 seconds in a T-peel configuration per ASTM D-1876 using an INSTRON tensile tester at a 30.5 cm per minute crosshead speed. The average peel force was recorded over a 5.08 cm peel distance for each test Three to five replicates were run and averaged together and are reported in grams/cm.

### Shear Adhesion Test

The degree to which a tape can adhere to itself can be measured in a shear mode in addition to a peel mode. The shear adhesion of a piece of tape laminated to itself was measured using the following procedure. Two 2.5 cm by 15.2 cm pieces of tape were laminated to each other adhesive side to adhesive side with 2.5 cm by 2.5 cm overlap. A 2 kilogram hard rubber roller was passed over the laminate once in each direction at 30.5 cm per minute to firmly adhere the two tapes to each other in the 2.5 cm by 2.5 cm overlap region. The two-tape laminate was clamped at one end and hung vertically in a 40°C oven for 15 minutes after which a 1 kilogram weight was attached to the loose bottom end of the laminate, generating a shear load at a 180° angle. The time that it took in minutes for the weight to drop at 40°C was recorded as a measure of the shear adhesion. Reported values are averages of 5 tests.

### Loop Tack Self-Adhesion Test

The ability of a tape to adhere to itself with short dwell time, sometimes referred to as "quick-stick," was measured using ASTM D-6195 Method A. The 2.5 cm by 2.5 cm test area of the stainless steel (#304) fixture was covered with a 2.5 cm by 2.5 cm piece (adhesive side up) of the same tape as used for the loop, such that the adhesive to adhesive bond strength was measured. The average peak force in grams/cm² required to remove the loop from the adhesive-covered steel fixture was recorded. Reported values are averages of 3 replicates.

### Loop Tack Steel-Adhesion Test

The ability of a tape to adhere to a test substrate with short dwell time, sometimes referred to as "quick-stick," was measured using ASTM D-6195 Method A. The 2.5 cm by 2.5 cm test area of the stainless steel (#304) fixture was left bare, such that the adhesive to steel bond strength was measured. A 5 second dwell or delay period was used after the upper crosshead reached the minimum position. The average peak force in grams/cm² required to remove the loop from the steel fixture was recorded. Reported values are averages of 3 replicates.

### Thumb Tack

Test specimens of the adhesives were evaluated for their degree of tack by performing a Thumb Tack test using the following procedure. Adhesive specimens were tested for "thumb tack" by placing an individual's washed and dried thumb on an exposed adhesive surface. The amount of adhesion of the tape to the surface of the thumb is rated using a qualitative assessment of "finger appeal" and assigned a value of 1 through 5 where 1= tack free, 2 = poor tack, 3 = medium tack, 4 = good tack, and 5 = excellent tack. On this scale, "Scotch" "Magic" transparent tape commercially available from 3M Company has a rating of 5. Reported values are averages of 3 replicates.

### Refastenability

To demonstrate that tapes of the present invention can be repeatedly refastened to themselves in a closure system without significant loss of peel force, the T-Peel Self-Adhesion test described above was performed on two pieces of tape having the same adhesive composition using the compositions described in Examples 3 and 9 herein. Two separate pieces of tape were tested in the same manner as described above in the T-Peel Self-Adhesion test except a 5-minute dwell time after roll-down was used instead of 10 seconds (5-minute dwell T-Peel). The same two pieces of tape were then readhered (i.e., laminated to each other adhesive side to adhesive side using a 100-gram roller) and tested again in the same manner as described in the T-Peel Self-Adhesion test with a 5-minute dwell time after roll-down instead of 10 seconds (1^{st} refastening T-Peel). The same two pieces of tape were once again readhered (i.e., laminated to each other adhesive side to adhesive side using a 100-gram roller) and tested in the same manner as described in the T-Peel Self-Adhesion test with a 5-minute dwell time after roll-down instead of 10 seconds (2nd refastening T-Peel). To demonstrate that the adhesion level does not build to significantly higher levels over time, two fresh pieces of tape having the same adhesive composition using the compositions described in Examples 3 and 9, were tested again in the same manner as described in the T-Peel Self-Adhesion test with a 10-second dwell time after roll-down instead of 5 minutes (Initial T-Peel Self). The results are reported in Table 10 below in grams/cm and are the averages of 2 replicates.

### Materials

The following materials were used in the preparation of the examples and comparative examples below.

### Elastomers:

| | |
|---|---|
| KRATON D-1107 | Styrene-Isoprene-Styrene (SIS) thermoplastic elastomer containing 14% styrene, available from Kraton Polymers U.S.LLC, Houston, TX. |
| KRATON G-1657 | Styrene-Ethylenebutylene-Styrene (SEBS) thermoplastic elastomer containing 13% styrene, available from Kraton Polymers U.S.LLC, Houston, TX. |
| Europrene SOLT 166 | Styrene-Butadiene-Styrene (SBS) thermoplastic elastomer containing 30% styrene, available from Polimeri Europa, Bayton, TX. |
| KRATON D-1124 | Radial Styrene-Isoprene-Styrene thermoplastic elastomer containing 18% styrene, available from Kraton Polymers U.S.LLC, Houston, TX. |
| KRATON D-1122 | SBS thermoplastic elastomer containing 37% styrene, available from Kraton Polymers U.S.LLC, Houston, TX. |
| KRATON D-1114 | SIS thermoplastic elastomer containing 18% styrene, available from Kraton Polymers U.S.LLC, Houston, TX. |
| KRATON D-1118 | SBS thermoplastic elastomer containing 31% styrene, available from Kraton Polymers U.S.LLC, Houston, TX. |
| NIPOL DN-1201L | Acrylonitrile-butadiene-isoprene polymer from Zeon Chemicals, Louisville, KY. |
| NIPOL DN-401L | Butadiene-acrylonitrile polymer (19% acrylonitrile) from Zeon Chemicals, Louisville, KY. |
| NIPOL DN-3635 | Butadiene-acrylonitrile polymer (36% acrylonitrile) from Zeon Chemicals, Louisville, KY. |

### Tackifiers:

| | |
|---|---|
| ESCOREZ 1310 | Aliphatic hydrocarbon tackifying resin, available from Exxon Chemical Co., Houston, TX. |
| ZONAREZ A-25 | Alpha-pinene tackifying resin, available from Arizona Chemical, Jacksonville, FL. |
| SYLVARES TR 1100 | Aliphatic polyterpene tackifying resin, available from Arizona Chemical, Jacksonville, FL. |
| PICCOLYTE A135 | Alpha-pinene tackifying resin, available from Eastman Chemical, Kingsport, TN |
| ESCOREZ 5380 | Hydrogenated cycloaliphatic hydrocarbon tackifying resin, available from Exxon Chemical Co., Houston, TX. |
| ESCOREZ 2510 | Aromatic modified aliphatic tackifying resin, available from Exxon Chemical Co., Houston, TX. |
| ESCOREZ 2520 | Liquid aliphatic/aromatic modified tackifing resin, available from Exxon Chemical Co., Houston, TX. |
| ESCOREZ 2101 | Aliphatic/aromatic hydrocarbon tackifying resin, available from Exxon Chemical Co., Houston, TX. |
| WINGTACK Plus | C5 aliphatic aromatically modified tackifying resin, available from Goodyear Chemical, Akron, OH. |
| WINGTACK Extra | C5 aliphatic aromatically modified tackifying resin, available from Goodyear Chemical, Akron, OH. |
| WINGTACK 10 | Liquid C5 aliphatic tackifying resin, available from Goodyear Chemical, Akron, OH. |

### Waxes:

| | |
|---|---|
| POLYWAX 2000 | Ethylene homopolymer wax (2000 Mn molecular weight) available from Baker Petrolite Corp., Sugar Land, TX. |
| POLYWAX 1000 | Ethylene homopolymer wax (1000 Mn molecular weight) available from Baker Petrolite Corp., Sugar Land, TX. |
| POLYWAX 500 | Ethylene homopolymer wax (500 Mn molecular weight) available from Baker Petrolite Corp., Sugar Land, TX. |
| PETROLITE EP-1100 | Ethylene-propylene copolymer wax (1100 Mn molecular weight) available from Baker Petrolite Corp., Sugar Land, TX. |
| POLYFLO 200 | Ethylene homopolymer wax available from Moore & Munger, Inc., Shelton, CT. |
| R-2551 1 | Fully refined paraffin wax available from Moore & Munger, Inc., Shelton, CT. |
| PARAFLINT H-4 | Synthetic paraffin, Fischer -Tropsch wax (750 Mn molecular weight) available from Moore & Munger, Inc., Shelton, CT. |

### Other Components:

| | |
|---|---|
| BENZOFLEX 352 | 1,4 cyclohexane dimethanol dibenzoate solid plasticizer, available from Velsicol Chemical Corp. Rosemont, IL. |
| SHELLFLEX 371 | Naphthenic oil, available from Shell Lubricants, Houston, TX. |
| NA 601 | Polyethylene, available from Equistar Chemicals, LP, Houston, TX. |
| IRGANOX 1076 | Antioxidant, available from Ciba Specialty Chemicals, Tarrytown, NY. |

### Backings:

A: 60 grams/square meter (g/m²) TiO₂ pigmented polypropylene homopolymer film having a surface roughness of approximately 50RA on both sides.
B: Dalex 1050 polycoated nonwoven, 50 g/m² polypropylene spunbond polycoated with a 28 g/m² polyolefin, available from Don & Low, Scotland.
C: 1.5 oz/yd² Spunbonded PP Non-woven, available from PGI, North Charleston, South Carolina.

Examples 1-12 and Comparative Examples C1 and C2, C9-C10 and C12

The non-tacky adhesives of Examples 1-12 and Comparative Examples C1-C2, C9-C10, and C12 were prepared using a 30 mm co-rotating, twin-screw extruder (42 L/D, Werner Pfleiderer). The extruder had six heating zones respectively set at 138, 149, 153, 156, 158 and 161°C. The adhesive components were fed at a rate so as to obtain a total throughput of the non-tacky adhesive composition of 0.82 kilograms per hour (kg/hr). The tackifier and the wax where each fed as a molten stream using a Zenith pump to meter the material. The base rubber component, combined with 1 part per hundred parts rubber of anti-oxidant, was metered using a gravimetric hopper feeder. The stabilized rubber was then metered into the feed zone of the extruder, followed by the addition of the molten tackifier in the first heating zone (set at 138°C). The molten wax was injected into the feed port of the second heating zone set at 149°C. The adhesive was coated onto various backings using a drop die set at 163°C. The backing was wrapped around a chill roll to support the material during coating. The adhesive coating was controlled to a coating weight of 32-48 grams per square centimeter (g/m²) and is reported in the tables below.

### Comparative Examples C7, C8, C11, C13 & C14

Comparative Examples C7, C8, C11, C13, and C14 were prepared using the following procedure. A melt mixing device was used to process the comparative example compositions, which were subsequently coated by extrusion of the molten compositions through a drop die onto the desired backing. The melt mixing device consisted of a barrel equipped with a static mixing element and heater. The device was heated to 171°C. The tackifiers and elastomers were added to the device and brought up to temperature (171°C) for approximately 10 minutes. One part anti-oxidant was added based on 100 parts of the elastomer to provide stability to the composition. When the components reached approximately 171°C, the composition was circulated through the static mixer for 30 cycles to provide a homogeneous molten composition. The wax component was then added and the composition processed through the static mixing element for another 30 cycles to provide a molten composition ready for coating. The coating was carried out by pumping the composition through a drop die set at a temperature of 160°C using the desired backing. The adhesive coating weight was varied and is reported in the tables below.

### Comparative Examples C3-C6 and C15-C17

The adhesive compositions of Comparative Examples C3-C6 were prepared by dissolving the components in toluene at a 35:65 solids:solvent ratio. Comparative Examples C15-C17 were prepared by dissolving the components in MEK at a 25: 75 solids:solvent ratio. One part per hundred parts of antioxidant was added to the solution. The adhesive solutions were coated onto backing 'A'. The adhesive coating was controlled to a coating weight of 38 g/m².

Table 1 below shows several non-tacky adhesive compositions of the invention based on KRATON D-1107 rubber with different tackifiers and waxes. Also shown are comparative examples showing high molecular weight waxes, high percentages of wax and no wax at all. Numbers in the Table are shown as parts per hundred parts of base rubber and also as percentages (shown in parentheses) of the individual components based on the entire composition. The testing results of the compositions are shown in Table 6.

**Table 1**

| Examples | | | | | | | | |
|---|---|---|---|---|---|---|---|---|
| Component | 1 | 2 | 3 | 4 | 5 | C1 | C2 | C3 |
| KRATON D-1107 | 100 | 100 | 100 | 100 | 100 | 100 | 100 | 100 |
| | (40) | (35) | (40) | (40) | (32) | (40.5) | (40) | (50) |
| ESCOREZ 1310 | 100 | 100 | 100 | 75 | 100 | 100 | 100 | |
| | (40) | (35) | (40) | (30) | (32) | (40.5) | (40) | |
| WINGTACK 10 | | | | 25 | | | | |
| | | | | (10) | | | | |
| ZONAREZ A25 | | | | | | | | 96 |
| | | | | | | | | (48) |
| SYLVARES TR1100 | | | | | | | | 4 |
| | | | | | | | | (2) |
| POLYWAX 2000 | | | | | | 46 | 50 | |
| | | | | | | (19) | (20) | |
| POLYWAX 1000 | 50 | | | 50 | | | | |
| | (20) | | | (20) | | | | |
| POLYWAX 500 | | 86 | | | 112 | | | |
| | | (30) | | | (36) | | | |
| PETROLITE EP-1100 Wax | | | 50 | | | | | |
| | | | (20) | | | | | |
| IRGANOX 1076 | 1 | 1 | 1 | 1 | 1 | 1 | 1 | 1 |
| Backing Type | A | A | A | A | A | A | A | A |
| Coating Weight (g/m²) | 32 | 32 | 32 | 32 | 32 | 32 | 32 | 36 |

Table 2 below shows several non-tacky adhesive compositions of the invention based on KRATON D-1107 rubber coated onto different backings. Also shown is a Comparative Example (C4) showing a known non-tacky adhesive formulation (U.S. Pat. No. 5,389,438, Example 6). Numbers in the Table are shown as parts per hundred parts of base rubber and also as percentages (shown in parentheses) of the individual components based on the entire composition. The testing results of the compositions are shown in Table 7.

**Table 2**

| Examples | | | | | |
|---|---|---|---|---|---|
| Component | 6 | 7 | 8 | 9 | C4 |
| KRATOND-1107 | 100 | 100 | 100 | 100 | 100 |
| | (40) | (44) | (44) | (56) | (80) |
| WINGTACK Plus | 100 | 75 | 75 | 40 | 16.25 |
| | (40) | (33) | (33) | (22) | (13) |
| POLYWAX 1000 | 50 | 50 | 50 | 40 | |
| | (20) | (23) | (23) | (22) | |
| SHELLFLEX 371 | | | | | 8.75 |
| | | | | | (7) |
| IRGANOX 1076 | 1 | 1 | 1 | 1 | 1 |
| Backing Type | B | B | C | B | A |
| Coating Weight (g/m²) | 32 | 32 | 48 | 32 | 36 |

Table 3 below shows several non-tacky adhesive compositions of the invention based on several different KRATON rubbers. A pure rubber without any tackifiers and/or waxes is shown as comparative example C5. Also shown is a Comparative Example C6, showing a known adhesive formulation (U.S. Pat. No. 5,019,072, Example 2 using Escorez 5380 tackifier in place of Res D-2084 as listed in the patent, due to unavailability of the Res D-2084 material) that is described as self-bonding to itself. Also shown are Comparative Examples C7 and C8, showing known adhesive formulations (U.S. Pat. No. 6,261,278, Examples 2 and 6) that have self-adhesive properties when bonded to themselves. Also shown is a Comparative Example C9, showing a wax level below the claimed level. Numbers in the table are shown as parts per hundred parts of base rubber and also as percentages (shown in parentheses) of the individual components based on the entire composition. The testing results of the compositions are shown in Table 8.

**Table 3**

| Examples | | | | | | | | |
|---|---|---|---|---|---|---|---|---|
| Component | 10 | 11 | 12 | C5 | C6 | C7 | C8 | C9 |
| KRATON G-1657 | 100 | | | 100 | 100 | 100 | 100 | 100 |
| | (45) | | | (100) | (70) | (90) | (60) | (47.6) |
| KRATON D-1114 | | 100 | | | | | | |
| | | (36.4) | | | | | | |
| KRATON D-1118 | | | 100 | | | | | |
| | | | (37) | | | | | |
| ESCOREZ 1310 | 100 | 100 | | | | | | 100 |
| | (45) | (36.4) | | | | | | (47.6) |
| PICCOLYT E 135 | | | 110 | | | | | |
| | | | (41) | | | | | |
| ESCOREZ 5380 | | | | | 43 | | | |
| | | | | | (30) | | | |
| POLYWAX 1000 | 23 | 75 | 40 | | | | | 10 |
| | (10) | (27.2) | (15) | | | | | (4.8) |
| NA-601 | | | | | | 11 | 67 | |
| | | | | | | (10) | (40) | |
| IRGANOX 1076 | 1 | 1 | 1 | 1 | 1 | 1 | 1 | 1 |
| Backing Type | A | A | A | A | A | A | A | A |
| Coating Weight (g/m²) | 32 | 32 | 32 | 36 | 36 | 41 | 29 | 32 |

Table 4 below shows several comparative example adhesive compositions. A bookbinding hot melt adhesive incorporating a high amount of solid plasticizer is shown as Comparative Examples C11 (U.S. Pat. No. 6,034,159 (Example 5), with Wingtack Extra tackifier used in place of the Unitac R-100Lt). Also shown is Comparative Example C13, showing an adhesive formulation (U.S. Pat. No. 5,275,589, sample 3, with SOLT168 used in place of SOLT166 and with ESCOREZ 2510 tackifier used in place of the ESCOREZ 149-A, Polyflo 200 in place of the ESCOMER H101, and Parafin wax 2551 in place of 6973 ) that is described as non-pressure sensitive. Also shown is Comparative Example C14, showing a known adhesive formulation (U.S. Pat. No. 4,942,195, sample A with ESCOREZ 2101 tackifier used in place of ESCOREZ 7312). Numbers in the Table are shown as parts per hundred parts of base rubber and also as percentages (shown in parentheses) of the individual components based on the entire composition. The testing results of the compositions are shown in Table 9.

**Table 4**

| Comparative Examples | | | | | |
|---|---|---|---|---|---|
| Component | C10 | C11 | C12 | C13 | C14 |
| KRATON D-1107 | 100 | | | | |
| | (33) | | | | |
| KRATON G-1657 | | | 100 | | |
| | | | (33.3) | | |
| KRATON D-1124 | | 100 | | | |
| | | (35) | | | |
| KRATON D-1122 | | | | | 100 |
| | | | | | (35) |
| Europrene SOLT | | | | 100 | |
| 166 | | | | (19) | |
| ESCOREZ 1310 | 100 | | 100 | | |
| | (33) | | (33.3) | | |
| ESCOREZ 2510 | | | | 234 | |
| | | | | (44.4) | |
| ESCOREZ 2520 | | | | 50 | |
| | | | | (9.5) | |
| ESCOREZ 2101 1 | | | | | 100 |
| | | | | | (35) |
| WINGTACK 10 | | | | 90 | |
| | | | | (17.1) | |
| WINGTACK Extra | | 106 | | | |
| | | (37) | | | |
| POLYWAX 1000 | 30 | | 30 | | |
| | (10) | | (10) | | |
| Parafin wax R-2551 | | | | 20.5 | 71.4 (25) |
| | | | | (3.9) | |
| POLYFLO 200 | | | | 30 (5.7) | |
| PARAFLINT H-4 | | 57 | | | |
| | | (20) | | | |
| BENZOFLEX 352 | 65 | 14 | 70 | | |
| | (21) | (5) | (23.4) | | |
| IRGANOX 1076 | 1 | 1 | 1 | 1 | 1 |
| Backing Type | A | A | A | A | A |
| Coating Weight (g/m²) | 32 | 64 | 32 | 236 | 336 |

Table 5 below shows three comparative non-tacky compositions based on the butadiene-acrylonitrile rubbers described in U.S. Pat. No. 5,888,335. Numbers in the Table are shown as parts per hundred parts of base rubber and also as percentages (shown in parentheses) of the individual components based on the entire composition. The testing results of the compositions are shown in Table 9.

**Table 5**

| Comparative Examples | | | |
|---|---|---|---|
| Component | C15 | C16 | C17 |
| NIPOL DN-401L | 100 | | |
| | (100) | | |
| NIPOL DN-1201L | | 100 | |
| | | (100) | |
| NIPOL DN-3635 | | | 100 |
| | | | (100) |
| Backing Type | B | B | B |
| Coating Weight (g/m²) | 44 | 48 | 48 |

**Table 6**

| Example | T-Peel Self-Adhesion (g/cm) | Loop Tack Self-Adhesion (g/cm²) | Loop Tack Steel-Adhesion (g/cm²) | Shear Adhesion (minutes) | Thumb Tack |
|---|---|---|---|---|---|
| 1 | 1150 | 491 | 0 | >900 | 2 |
| 2 | 481 | | 0.3 | >900 | 2 |
| 3 | 715 | | 5 | >900 | 2 |
| 4 | 815 | | 7 | >900 | 2 |
| 5 | 72 | | | | |
| C1* | | | | | |
| C2* | | | | | |
| C3 | 113 | 107 | 64 | 8 | 5 |

| | | | | | |
|---|---|---|---|---|---|
| *Could not be extruded into a film for testing. | | | | | |

**Table 7**

| Example | T-Peel Self-Adhesion (g/cm) | Loop Tack Self-Adhesion (g/cm²) | Loop Tack Steel-Adhesion (g/cm²) | Shear Adhesion (minutes) | Thumb Tack |
|---|---|---|---|---|---|
| 6 | 389 | | 0 | 250 | 2 |
| 7 | 481 | | 0 | 440 | 2 |
| 8 | 124 | | 0 | 194 | 2 |
| 9 | 154 | | 0.2 | 190 | 2 |
| C4 | 61 | | 44 | >900 | 4 |

**Table 8**

| Example | T-Peel Self-Adhesion (g/cm) | Loop Tack Self-Adhesion (g/cm²) | Loop Tack Steel-Adhesion (g/cm²) | Shear Adhesion (minutes) | Thumb Tack |
|---|---|---|---|---|---|
| 10 | 365 | 143 | 18 | >900 | 2 |
| 11 | 398 | | 0.2 | >900 | 2 |
| 12 | 136 | | 5 | >900 | 2 |
| C5 | 97 | 8 | 0 | >900 | 1 |
| C6 | 113 | 31 | 2 | >900 | 2 |
| C7 | 37 | | 0.5 | | 1 |
| C8 | 46 | | | | 1 |
| C9 | 377 | | 93 | | |

**Table 9**

| Example | T-Peel Self-Adhesion (g/cm) | Loop Tack Self-Adhesion (g/cm²) | Loop Tack Steel-Adhesion (g/cm²) | Shear Adhesion (minutes) | Thumb Tack |
|---|---|---|---|---|---|
| C10 | 34 | | 0 | 3 | 2 |
| C11 | 39 | | 2 | | 2 |
| C12 | 61 | | | | |
| C13 | 1 | | 0 | | 1 |
| C14 | 0 | | 0.5 | | 1 |
| C15 | 386 | | 81 | | |
| C16 | 169 | 69 | 39 | | |
| C17 | 67 | | 13 | | |

Table 10 demonstrates the refastenability of two samples (Examples 3 and 9). These results show that even after two refastening steps, these embodiments of the self-adhering adhesive demonstrated good T-Peel results.

**Table 10**

| Test | Example 3 | Example 9 |
|---|---|---|
| Initial T-Peel Self | 612 | 300 |
| Adhesion (g/cm) | | |
| 5-Minute Dwell T-Peel | 535 | 343 |
| Self Adhesion (g/cm) | | |
| 1^{st} Refastening T-Peel | 543 | 272 |
| Self Adhesion (g/cm) | | |
| 2^{nd} Refastening T-Peel | 569 | 212 |
| Self Adhesion (g/cm) | | |

Various modifications and alterations to this invention will become apparent to those skilled in the art without departing from the scope of this invention. It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. A method for closing a closure system comprising:
at least two opposing closure elements that are capable of overlapping to form an adhesive closure; and
a self-adhering adhesive composition on each of the closure elements;
wherein the self-adhering adhesive composition comprises:
50 wt-% to 90 wt-% of an adhesive elastomeric material comprising a nonpolar elastomer;
10 wt-% to 40 wt-% of a wax;
0 to less than 5 wt-% of a plasticizer;
wherein the percentages are based on the total weight of the adhesive elastomeric material plus wax plus optional plasticizer; and
wherein the self-adhering adhesive composition has a T-peel of at least 100 grams per centimeter and a loop tack to steel of no greater than 40 grams per square centimeter,
wherein the T-peel is measured according to the T Peel Self-Adhesion Test and the loop tack to steel is measured according to the Loop Tack Steel-Adhesion Test,
wherein the method comprises the step of bringing the opposing closure elements into contact to yield an adhesive closure.

2. The method of claim 1 wherein the self-adhering adhesive composition has a shear adhesion value of at least 100 minutes, wherein the shear adhesion value is measured according to the Shear Adhesion Test.

3. The method of claim 1 wherein the self-adhering adhesive composition has a loop tack self-adhesion of at least 120 grams per square centimeter, wherein the loop tack self-adhesion is measured according to the Loop Tack Self-Adhesion Test.

4. The method of claim 1 wherein the self-adhering adhesive composition comprises 10 wt-% to 35 wt-% of a wax, based on the total weight of the adhesive elastomeric material plus wax plus optional plasticizer.

5. The method of claim 4 wherein the self-adhering adhesive composition comprises 10 wt-% to 30 wt-% of a wax, based on the total weight of the adhesive elastomeric material plus wax plus optional plasticizer and the adhesive elastomeric material comprises a block copolymer thermoplastic elastomer and/or a tackifying resin.

6. The method of claim 1 wherein the self-adhering adhesive composition further comprises a plasticizer.

7. The method of claim 1 wherein the wax has a molecular weight of less than 1500.

8. The method of claim 1 wherein the self-adhering adhesive composition has a T-peel of at least 100 grams per cm, a shear adhesion value of at least 100 minutes, a loop tack steel-adhesion of no greater than 40 grams per square centimeter, and a thumb tack of no greater than 3.

9. A bag comprising:
at least two regions of the bag having a layer of a self-adhering adhesive composition
disposed thereon at separate locations in a manner such that the regions are capable of overlapping to form an adhesive closure;
wherein the self-adhering adhesive composition comprises:
50 wt-% to 90 wt-% of an adhesive elastomeric material comprising a nonpolar elastomer;
10 wt-% to 40 wt-% of a wax;
0 to less than 5 wt-% of a plasticizer;
wherein the percentages are based on the total weight of the adhesive elastomeric material plus wax plus optional plasticizer; and
wherein the self-adhering adhesive composition has a T-peel of at least 100 grams per cm and a loop tack to steel of no greater than 40 grams per square centimeter, wherein the T-peel is measured according to the T Peel Self-Adhesion Test and the loop tack to steel is measured according to the Loop Tack Steel-Adhesion Test.

10. A banding system comprising:
at least two strips of a banding material with at least two regions of a self-adhering
adhesive composition disposed thereon at separate locations in a manner such that the regions of adhesive are capable of overlapping to form an adhesive closure
wherein the self-adhering adhesive composition comprises:
50 wt-% to 90 wt-% of an adhesive elastomeric material comprising a nonpolar elastomer;
10 wt-% to 40 wt-% of a wax;
0 to less than 5 wt-% of a plasticizer;
wherein the percentages are based on the total weight of the adhesive elastomeric material plus wax plus optional plasticizer; and
wherein the self-adhering adhesive composition has a T-peel of at least 100 grams per cm and a loop tack to steel of no greater than 40 grams per square centimeter, wherein the T-peel is measured according to the T Peel Self-Adhesion Test and the loop tack to steel is measured according to the Loop Tack Steel-Adhesion Test.

11. An apparel comprising:
a closure system comprising:
at least two opposing closure elements that are capable of overlapping to form an adhesive closure; and
a self-adhering adhesive composition on each of the closure elements;
wherein the self-adhering adhesive composition comprises:
50 wt-% to 90 wt-% of an adhesive elastomeric material comprising a nonpolar elastomer;
10 wt-% to 40 wt-% of a wax;
0 to less than 5 wt-% of a plasticizer;
wherein the percentages are based on the total weight of the adhesive elastomeric material plus wax plus optional plasticizer; and
wherein the self-adhering adhesive composition has aT-peel of at least 100 grams per centimeter and a loop tack to steel of no greater than 40 grams per square centimeter,
wherein the T-peel is measured according to the T Peel Self-Adhesion Test and the loop tack to steel is measured according to the Loop Tack Steel-Adhesion Test, wherein the closure system is to be used in close proximity to the wearer.

12. The apparel of claim 11, which is a diaper, incontinence device, surgical gown, hat, bootie, clean room garment, ankle band or wrist band.

## Patentansprüche

1. Verfahren zum Verschließen eines Verschlusssystems umfassend:
mindestens zwei gegenüberliegende Verschlusselemente, die in der Lage sind, sich zu überlappen, um einen Klebeverschluss zu bilden; und
eine selbsthaftende Klebstoffzusammensetzung auf jedem Verschlusselement;
wobei die selbsthaftende Klebstoffzusammensetzung umfasst:
50 Gew.-% bis 90 Gew.-% eines klebenden elastomeren Materials, das ein nichtpolares Elastomer umfasst;
10 Gew.-% bis 40 Gew.-% von einem Wachs;
0 bis weniger als 5 Gew.-% von einem Weichmacher;
wobei die prozentualen Anteile auf dem Gesamtgewicht des klebenden elastomeren Materials zuzüglich Wachs zuzüglich des fakultativen Weichmachers basieren; und
wobei die selbsthaftende Klebstoffzusammensetzung einen T-Peel-Wert von mindestens 100 Gramm pro Zentimeter und einen Loop-Tack-Wert zu Stahl von nicht größer als 40 Gramm pro Quadratzentimeter aufweist, wobei der T-Peel-Wert gemäß dem T-Peel-Selbstadhäsionstest und der Loop-Tack-Wert zu Stahl gemäß dem Loop-Tack Stahladhäsionstest gemessen wird,
wobei das Verfahren den Schritt des Inkontaktbringens der gegenüberliegenden Verschlusselemente zum Erhalten eines Klebeverschlusses umfasst.

2. Verfahren nach Anspruch 1, wobei die selbsthaftende Klebstoffzusammensetzung einen Wert der Scheradhäsion von mindestens 100 Minuten aufweist, wobei der Wert der Scheradhäsion gemäß dem Scheradhäsionstest gemessen wird.

3. Verfahren nach Anspruch 1, wobei die selbsthaftende Klebstoffzusammensetzung einen Loop-Tack-Wert der Selbstadhäsion von mindestens 120 Gramm pro Quadratzentimeter aufweist, wobei der Wert der Loop-Tack Selbstadhäsion gemäß dem Loop-Tack Selbstadhäsionstest gemessen wird.

4. Verfahren nach Anspruch 1, wobei die selbsthaftende Klebstoffzusammensetzung 10 Gew.-% bis 35 Gew.-% von einem Wachs umfasst, basierend auf dem Gesamtgewicht des klebenden elastomeren Materials zuzüglich Wachs und zuzüglich des fakultativen Weichmachers.

5. Verfahren nach Anspruch 4, wobei die selbsthaftende Klebstoffzusammensetzung 10 Gew.-% bis 30 Gew.-% von einem Wachs aufweist, basierend auf dem Gesamtgewicht des klebenden elastomeren Materials zuzüglich Wachs und zuzüglich des fakultativen Weichmachers und das klebende elastomere Material ein thermoplastisches Blockcopolymer-Elastomer und/oder ein klebrig machendes Harz umfasst.

6. Verfahren nach Anspruch 1, wobei die selbsthaftende Klebstoffzusammensetzung ferner einen Weichmacher umfasst.

7. Verfahren nach Anspruch 1, wobei das Wachs ein Molekulargewicht von weniger als 1.500 aufweist.

8. Verfahren nach Anspruch 1, wobei die selbsthaftende Klebstoffzusammensetzung einen T-Peel-Wert von mindestens 100 Gramm pro cm, einen Scheradhäsionswert von mindestens 100 Minuten, einen Loop-Tack der Stahladhäsion von nicht größer als 40 Gramm pro Quadratzentimeter und eine Anfassklebkraft von nicht größer als 3 aufweist.

9. Beutel umfassend:
mindestens zwei Bereiche von dem Beutel, die eine Schicht von einer selbsthaftenden Klebstoffzusammensetzung aufweisen, welche an getrennten Stellen in einer derartigen Weise darauf verteilt ist, dass diese Bereiche in der Lage sind, sich zu überlappen, um einen Klebeverschluss zu bilden;
wobei die selbsthaftende Klebstoffzusammensetzung umfasst:
50 Gew.-% bis 90 Gew.-% eines klebenden elastomeren Materials, das ein nichtpolares Elastomer umfasst;
10 Gew.-% bis 40,Gew.-% von einem Wachs;
0 bis weniger als 5 Gew.-% von einem Weichmacher;
wobei die prozentualen Anteile auf dem Gesamtgewicht des klebenden elastomeren Materials zuzüglich Wachs zuzüglich des fakultativen Weichmachers basieren; und
wobei die selbsthaftende Klebstoffzusammensetzung einen T-Peel-Wert von mindestens 100 Gramm pro cm und einen Loop-Tack-Wert zu Stahl von nicht größer als 40 Gramm pro Quadratzentimeter aufweist, wobei der T-Peel-Wert gemäß dem T-Peel-Selbstadhäsionstest und der Loop-Tack-Wert zu Stahl gemäß dem Loop-Tack Stahladhäsionstest gemessen wird.

10. Bandsystem umfassend:
mindestens zwei Streifen von einem Bandmaterial mit mindestens zwei Bereichen einer selbsthaftenden Klebstoffzusammensetzung, welche an getrennten Stellen in einer derartigen Weise darauf verteilt ist, dass die Bereiche in der Lage sind, sich zu überlappen, um einen Klebeverschluss zu bilden;
wobei die selbsthaftende Klebstoffzusammensetzung umfasst:
50 Gew.-% bis 90 Gew.-% eines klebenden elastomeren Materials, das ein nichtpolares Elastomer umfasst;
10 Gew.-% bis 40 Gew.-% von einem Wachs;
0 bis weniger als 5 Gew.-% von einem Weichmacher;
wobei die prozentualen Anteile auf dem Gesamtgewicht des klebenden elastomeren Materials zuzüglich Wachs zuzüglich des fakultativen Weichmachers basieren; und
wobei die selbsthaftende Klebstoffzusammensetzun einen T-Peel-Wert von mindestens 100 Gramm pro Zentimeter und einen Loop-Tack-Wert zu Stahl von nicht größer als 40 Gramm pro Quadratzentimeter aufweist, wobei der T-Peel-Wert gemäß dem T-Peel-Selbstadhäsionstest und der Loop-Tack-Wert zu Stahl gemäß dem Loop-Tack Stahladhäsionstest gemessen wird.

11. Kleidungsstück umfassend:
ein Verschlusssystem umfassend:
mindestens zwei gegenüberliegende Verschlusselemente, die in der Lage sind, sich zu überlappen, um einen Klebeverschluss zu bilden; und
eine selbsthaftende Klebstoffzusammensetzung auf jedem der Verschlusselemente;
wobei die selbsthaftende Klebstoffzusammensetzung umfasst:
50 Gew.-% bis 90 Gew.-% eines klebenden elastomeren- Materials, das ein nichtpolares Elastomer umfasst;
10 Gew.-% bis 40 Gew.-% von einem Wachs;
0 bis weniger als 5 Gew.-% von einem Weichmacher;
wobei die prozentualen Anteile auf dem Gesamtgewicht des klebenden elastomeren Materials zuzüglich Wachs zuzüglich des fakultativen Weichmachers basieren; und
wobei die selbsthaftende Klebstoffzusammensetzung einen T-Peel-Wert von mindestens 100 Gramm pro Zentimeter und einen Loop-Tack-Wert zu Stahl von nicht größer als 40 Gramm pro Quadratzentimeter aufweist, wobei der T-Peel-Wert gemäß dem T-Peel-Selbstadhäsionstest und der Loop-Tack-Wert zu Stahl gemäß dem Loop-Tack Stahladhäsionstest gemessen wird, wobei das Verschlusssystem in unmittelbarer Nähe des Trägers zu verwenden ist.

12. Kleidungsstück nach Anspruch 11, welches eine Windel, eine Inkontinenz-Vorrichtung, ein Operationskittel, ein Hut, ein Babyschuh, eine Reinraumbekleidung, ein Knöchelband oder ein Handgelenkband ist.

## Revendications

1. Méthode de fermeture d'un système de fermeture comprenant :
au moins deux éléments de fermeture opposés qui sont capables d'être superposés pour former une fermeture adhésive ; et
une composition adhésive autocollante sur chacun des éléments de fermeture ;
dans laquelle la composition adhésive autocollante comprend :
50 % en poids à 90 % en poids d'un matériau élastomère adhésif comprenant un élastomère non polaire ;
10 % en poids à 40 % en poids d'une cire ;
0 à moins de 5 % en poids d'un plastifiant ;
les pourcentages étant exprimés relativement au poids total du matériau élastomère adhésif plus la cire plus le plastifiant facultatif ; et
dans laquelle la composition adhésive autocollante a une valeur T-Peel d'au moins 100 grammes par centimètre et une valeur loop tack sur l'acier qui n'est pas supérieure à 40 grammes par centimètre carré, la valeur T-Peel étant mesurée selon l'Essai de détermination de l'auto-adhésivité T-Peel et la valeur loop tack sur l'acier étant mesurée selon l'Essai de détermination de l'adhésivité Loop Tack sur l'acier,
la méthode comprenant l'étape qui consiste à mettre les éléments de fermeture opposés en contact pour produire une fermeture adhésive.

2. Méthode selon la revendication 1, dans laquelle la composition adhésive autocollante a une valeur de pouvoir adhésif tangentiel d'au moins 100 minutes, la valeur de pouvoir adhésif tangentiel étant mesurée selon l'Essai de détermination du pouvoir adhésif tangentiel.

3. Méthode selon la revendication 1, dans laquelle la composition adhésive autocollante a une valeur loop tack de l'auto-adhésivité d'au moins 120 grammes par centimètre carré, la valeur loop tack de l'auto-adhésivité étant mesurée selon l'Essai de détermination de l'auto-adhésivité Loop Tack.

4. Méthode selon la revendication 1, dans laquelle la composition adhésive autocollante comprend de 10 % en poids à 35 % en poids d'une cire, ce pourcentage étant exprimé relativement au poids total du matériau élastomère adhésif plus la cire plus le plastifiant facultatif.

5. Méthode selon la revendication 4, dans laquelle la composition adhésive autocollante comprend de 10 % en poids à 30 % en poids d'une cire, ce pourcentage étant exprimé relativement au poids total du matériau élastomère adhésif plus la cire plus le plastifiant facultatif, et le matériau élastomère adhésif comprend un élastomère thermoplastique qui est un copolymère à blocs et/ou une résine tackifiante.

6. Méthode selon la revendication 1, dans laquelle la composition adhésive autocollante comprend en outre un plastifiant.

7. Méthode selon la revendication 1, dans laquelle la cire a une masse moléculaire inférieure à 1 500.

8. Méthode selon la revendication 1, dans laquelle la composition adhésive autocollante a une valeur T-Peel d'au moins 100 grammes par centimètre, une valeur de pouvoir adhésif tangentiel d'au moins 100 minutes, une valeur loop tack sur l'acier qui n'est pas supérieure à 40 grammes par centimètre carré, et une adhésivité instantanée sur le pouce qui n'est pas supérieure à 3.

9. Poche comprenant :
au moins deux régions de la poche comportant une couche d'une composition adhésive autocollante disposée sur elles à des emplacements séparés de sorte que les régions soient capables d'être superposées pour former une fermeture adhésive ;
dans laquelle la composition adhésive autocollante comprend :
50 % en poids à 90 % en poids d'un matériau élastomère adhésif comprenant un élastomère non polaire ;
10 % en poids à 40 % en poids d'une cire ;
0 à moins de 5 % en poids d'un plastifiant ;
les pourcentages étant exprimés relativement au poids total du matériau élastomère adhésif plus la cire plus le plastifiant facultatif; et
dans laquelle la composition adhésive autocollante a une valeur T-Peel d'au moins 100 grammes par centimètre et une valeur loop tack sur l'acier qui n'est pas supérieure à 40 grammes par centimètre carré, la valeur T-Peel étant mesurée selon l'Essai de détermination de l'auto-adhésivité T-Peel et la valeur loop tack sur l'acier étant mesurée selon l'Essai de détermination de l'adhésivité Loop Tack sur l'acier.

10. Système de cerclage comprenant :
au moins deux bandes d'un matériau de cerclage comprenant au moins deux régions d'une composition adhésive autocollante disposée sur elles à des emplacements séparés de sorte que les régions d'adhésif soient capables d'être superposées pour former une fermeture adhésive ;
dans lequel la composition adhésive autocollante comprend :
50 % en poids à 90 % en poids d'un matériau élastomère adhésif comprenant un élastomère non polaire ;
10 % en poids à 40 % en poids d'une cire ;
0 à moins de 5 % en poids d'un plastifiant ;
les pourcentages étant exprimés relativement au poids total du matériau élastomère adhésif plus la cire plus le plastifiant facultatif ; et
dans lequel la composition adhésive autocollante a une valeur T-Peel d'au moins 100 grammes par centimètre et une valeur loop tack sur l'acier qui n'est pas supérieure à 40 grammes par centimètre carré, la valeur T-Peel étant mesurée selon l'Essai de détermination de l'auto-adhésivité T-Peel et la valeur loop tack sur l'acier étant mesurée selon l'Essai de détermination de l'adhésivité Loop Tack sur l'acier.

11. Vêtement comprenant :
un système de fermeture comprenant :
au moins deux éléments de fermeture opposés qui sont capables d'être superposés pour former une fermeture adhésive ; et
une composition adhésive autocollante sur chacun des éléments de fermeture ;
dans lequel la composition adhésive autocollante comprend :
50 % en poids à 90 % en poids d'un matériau élastomère adhésif comprenant un élastomère non polaire ;
10 % en poids à 40 % en poids d'une cire ;
0 à moins de 5 % en poids d'un plastifiant ;
les pourcentages étant exprimés relativement au poids total du matériau élastomère adhésif plus la cire plus le plastifiant facultatif; et
dans lequel la composition adhésive autocollante a une valeur T-Peel d'au moins 100 grammes par centimètre et une valeur loop tack sur l'acier qui n'est pas supérieure à 40 grammes par centimètre carré, la valeur T-Peel étant mesurée selon l'Essai de détermination de l'auto-adhésivité T-Peel et la valeur loop tack sur l'acier étant mesurée selon l'Essai de détermination de l'adhésivité Loop Tack sur l'acier,
dans lequel le système de fermeture est destiné à une utilisation dans le voisinage immédiat de la personne qui porte le dispositif.

12. Vêtement selon la revendication 11, lequel est une couche pour bébé, un dispositif d'incontinence, un habillage chirurgical, un chapeau, un chausson, un vêtement de salle propre, un bracelet porté à la cheville, ou un bracelet porté au poignet.
